Europäisches Patentamt

(19) European Patent Office

Office européen des brevets

(11) Numéro de publication : **0 055 652**
**B1**

(12) **FASCICULE DE BREVET EUROPÉEN**

(45) Date de publication du fascicule du brevet :
20.02.85

(51) Int. Cl.⁴ : **B 01 J 23/26**, C 07 C 17/20, C 07 C 19/08

(21) Numéro de dépôt : **81401980.8**

(22) Date de dépôt : **11.12.81**

(54) **Catalyseurs de fluoruration en phase gazeuse de dérivés chlorés aliphatiques, à base de microbilles d'oxyde de chrome, et procédés de fluoruration utilisant ces catalyseurs.**

(30) Priorité : 29.12.80 FR 8027659

(43) Date de publication de la demande :
07.07.82 Bulletin 82/27

(45) Mention de la délivrance du brevet :
20.02.85 Bulletin 85/08

(84) Etats contractants désignés :
BE DE FR GB IT NL

(56) Documents cités :
FR-A- 1 369 782
US-A- 3 258 500
US-A- 3 426 009
US-A- 3 978 145
Le dossier contient des informations techniques présentées postérieurement au dépôt de la demande et
ne figurant pas dans le présent fascicule.

(73) Titulaire : ATOCHEM
12/16, allée des Vosges
F-92400 Courbevoie (FR)

(72) Inventeur : Foulletier, Louis
5, chemin Croix-Berthet
F-69600 Oullins (FR)

(74) Mandataire : Rochet, Michel et al
ATOCHEM Département Propriété Industrielle Cedex
22
F-92091 Paris la Défense (FR)

# 0 055 652

## Description

La présente invention concerne des catalyseurs de fluoruration en phase gazeuse des dérivés chlorés aliphatiques par l'acide fluorhydrique anhydre, constitués de microbilles d'oxyde de chrome $Cr_2O_3$ obtenues par un procédé « sol-gel ». L'invention concerne également des procédés de fluoruration des dérivés chlorés aliphatiques mettant en jeu ces catalyseurs.

De très nombreux catalyseurs ont été proposés pour ces réactions de fluoruration par substitution d'atomes de fluor à des atomes de chlore. Ce sont le plus souvent des oxydes ou des halogénures de chrome, d'aluminium, de cobalt, de fer, de titane, de nickel, de cuivre, de palladium ou de zirconium, utilisés tels quels ou sur des supports divers comme les charbons actifs et les alumines.

Les catalyseurs au chrome se répartissent en trois grandes familles :

— les catalyseurs au fluorure de chrome $CrF_3$,
— les catalyseurs aux oxyfluorures de chrome,
— les catalyseurs à l'oxyde de chrome $Cr_2O_3$.

Des catalyseurs à l'oxyde de chrome sont décrits, par exemple, dans le brevet britannique n° 896 068 et dans le brevet des Etats-Unis n° 3 157 707. Ces catalyseurs sont obtenus par déshydratation d'hydroxyde de chrome ou par réduction à l'hydrogène de trioxyde de chrome déposé sur alumine activée. Le brevet français n° 1 369 782 décrit également des catalyseurs à l'oxyde de chrome, non supportés, obtenus par déshydratation d'hydroxyde de chrome et sous forme de granulés de 0,8 à 4,7 mm. Dans le brevet US-A-3 258 500 est revendiqué un catalyseur à base de $Cr_2O_3$, non supporté, présentant une dimension particulaire comprise entre 0,8 et 2 mm ; il est obtenu par réduction du trioxyde de chrome à l'éthanol, chauffage à l'ébullition jusqu'à précipitation d'un gel d'oxyde de chrome, séchage, puis déshydratation et activation par chauffage en atmosphère inerte à 400-600 °C.

D'autres brevets, comme les demandes japonaises publiées sous les numéros 74.046718 et 74.134610 enseignent la réduction du trioxyde de chrome par des aldéhydes ou par l'hydrazine.

Des catalyseurs à base d'oxydes de chrome noirs, obtenus par décomposition thermique, en présence d'air ou d'oxygène, du trioxyde de chrome, de l'hydroxyde de chrome ou du carbonate de chrone, sont décrits dans le brevet français n° 1 358 997.

Le brevet des Etats-Unis n° 3 978 145 montre que les catalyseurs à l'oxyde de chrome préparés antérieurement ne sont pas amorphes, mais présentent la structure cristalline d'un hydroxyde-oxyde de chrome $\gamma$-CrOOH orthorhombique. Il propose des catalyseurs à durée de vie améliorée constitués par la forme hexagonale de cet hydroxyde-oxyde de chrome.

Tous ces catalyseurs aux oxydes de chrome de l'art antérieur conviennent plus ou moins bien à la fluoruration en phase gazeuse des dérivés chlorés aliphatiques dans des réacteurs à lit fixe. Ils sont par contre très mal adaptés à la fluoruration dans les réacteurs à lit fluidisé, qui exigent des particules de forme régulière et de granulométrie homogène. Le simple broyage des catalyseurs, suivi d'un tamisage pour sélectionner les particules de taille convenable, donne des particules de forme irrégulière et conduit à des déchets importants de catalyseur.

La demanderesse a découvert que les catalyseurs de fluoruration en phase gazeuse s'empoisonnent principalement par la formation de goudrons à leur surface. L'emploi de réacteurs à lit fluidisé, provoquant l'abrasion des grains de catalyseur, permet d'éliminer ces goudrons et de maintenir l'activité du catalyseur. Celui-ci se consomme uniquement par attrition, et il n'est plus besoin d'arrêter l'installation pour recharger le réacteur en catalyseur.

Un autre inconvénient des catalyseurs connus à l'oxyde de chrome est leur faible résistance à la cristallisation, qui contribue à abréger leur durée de vie. La plupart des catalyseurs à l'oxyde de chrome de l'art antérieur ont également l'inconvénient de fournir un taux relativement élevé en isomères asymétriques, lorsqu'ils sont utilisés pour la préparation du tétrachlorodifluoréthane, du trichlorotrifluoréthane et du dichlorotétrafluoréthane. Il est souhaitable que la teneur en ces isomères asymétriques, plus sensibles à l'hydrolyse et donc plus corrosifs, soit la plus réduite possible.

La présente invention a pour objet la préparation de catalyseurs à l'oxyde de chrome amorphes, résistant à la cristallisation, particulièrement adaptés aux réacteurs à lit fluidisé, et fournissant des teneurs élevées en isomères symétriques des dérivés perchloro-fluorés de l'éthane à 2, 3 ou 4 atomes de chlore.

De tels catalyseurs sont obtenus sous forme de microbilles, ayant des diamètres compris entre 100 μm et 3 000 μm, et de préférence voisins de 300 μm. Ces microbilles sont préparées suivant le procédé « sol-gel », qui a fait l'objet d'études importantes sur les oxydes de thorium, de zirconium et d'uranium. Ces travaux sur le procédé « sol-gel » sont décrits en particulier dans l'article de J. M. FLETCHER, Chemistry and Industry, 13 janvier 1968, page 48. Il est également connu d'après les travaux de J. DUCLAUX (Bulletin de la Société Chimique de France, 1956 page 1289) que les hydroxydes de chrome peuvent être mis sous la forme de « sols ».

Le procédé de préparation des microbilles d'oxyde de chrome comprend quatre étapes :

a) formation d'un « sol » ou hémicolloïde d'hydroxyde de chrome ;

2

0 055 652

b) dispersion du « sol » sous forme de gouttelettes dans un solvant organique non miscible à l'eau ou seulement partiellement miscible à l'eau, et gélification à température élevée ;

c) lavage des microbilles obtenues à l'ammoniaque et à l'eau pour éliminer la majorité des anions ;

d) séchage des microbilles, suivi d'un traitement thermique à des températures de 100 à 500 °C pour développer leur activité catalytique.

La formation du « sol » d'hydroxyde de chrome peut être faite par tout moyen connu, mais il est préféré de former le « sol » à partir de solutions de sels de chrome, comme le chlorure ou le nitrate, qui sont partiellement neutralisées à l'ammoniaque et qui contiennent de l'hexaméthylènetétramine. Ce composé dégage une quantité supplémentaire d'ammoniaque à la température de gélification du « sol » et favorise l'obtention de microbilles bien sphériques. Le « sol » peut également être préparé par réduction au méthanol d'une solution aqueuse de trioxyde de chrome.

Le « sol » est ensuite dispersé dans un solvant non miscible à l'eau, tel qu'un hydrocarbure liquide ou un haloalcane, ou mieux dans un solvant partiellement miscible à l'eau, tels que les alcools contenant de 4 à 8 atomes de carbone.

Le solvant préféré est l'éthyl-2-hexanol.

Dans le cas des solvants non miscibles à l'eau, la gélification du « sol » dispersé en gouttelettes se fait par évaporation de l'eau. Dans le cas des solvants miscibles à l'eau, il est avantageux d'utiliser une colonne de gélification, telle que celles décrites dans l'article de P. HAAS, Industrial Engineering Chemistry, Product Research and Development, 5, 236 (Septembre 1966) et dans le certificat d'addition n° 88 004 au brevet français n° 1 418 499.

Le « sol » d'hydroxyde de chrome est injecté au sommet de la colonne au moyen d'un tube de faible diamètre, disposé concentriquement à l'intérieur d'un autre tube de plus grand diamètre, par où arrive, comme fluide moteur, le solvant partiellement miscible à l'eau, réchauffé à une température de 25 à 140 °C. Le diamètre de l'injecteur de « sol » et le débit du fluide moteur conditionnent le diamètre des gouttelettes dispersées et par suite le diamètre final des microbilles.

La colonne elle-même est maintenue à une température de 25 à 140 °C. Elle est parcourue de bas en haut, par un autre courant de solvant chaud, injecté vers la base de la colonne, et qui sort au sommet par un trop plein, après s'être enrichi en eau. Ce solvant est déshydraté par distillation-décantation et renvoyé à la base de la colonne.

Divers additifs, tels que des agents mouillants et des épaississants peuvent être ajoutés au « sol » et/ou au solvant, pour améliorer la sphéricité des microbilles. Il est parfois avantageux d'ajouter au « sol » 1 à 10 % de silice colloïdale par rapport aux matières sèches pour améliorer la tenue mécanique des microbilles. Cette silice est éliminée par l'acide fluorhydrique au cours des réactions de fluoruration, ce qui contribue aussi à augmenter la porosité du catalyseur.

A la base de la colonne, on recueille les microbilles de catalyseur dans un récepteur, maintenu de préférence à une température de 115 °C, pour éviter les agglomérations.

Les microbilles sont ensuite lavées pendant plusieurs heures à l'ammoniaque concentré, puis à l'eau.

Le séchage s'effectue à l'air, à une température d'environ 130 °C.

Les microbilles ainsi obtenues n'ont pratiquement aucune activité catalytique dans les réactions de fluoruration. Il faut les soumettre à un traitement thermique à environ 400 °C, pendant 1 à 72 heures, dans une atmosphère d'air ou de gaz inerte.

Ces microbilles d'oxyde de chrome ont une structure amorphe et résistent particulièrement bien à la cristallisation. Un catalyseur de ce type ayant fonctionné pendant près de 500 heures à 400 °C est encore totalement amorphe.

Les catalyseurs selon l'invention présentent une certaine sélectivité dans la fluoruration des dérivés chlorés aliphatiques. Ils conviennent particulièrement à la fluoruration des dérivés chlorés et chlorofluorés du méthane et de l'éthane et à la fluoruration de l'hexachloracétone. Ils sont moins bien adaptés à la fluoruration des chloronitriles.

Les exemples suivants, donnés à titre non limitatif, illustrent divers modes de préparation des microbilles d'oxyde de chrome et leur utilisation dans la catalyse en lit fluidisé de diverses réactions de fluoruration.

Exemple 1

Préparation des microbilles d'oxyde de chrome.

On prépare un « sol » d'hydroxyde de chrome en mélangeant du chlorure de chrome, de l'ammoniaque et de l'hexaméthylènetétramine en solution aqueuse, dans les proportions suivantes :

2    moles/l de chlorure de chrome
1,65 mole/l d'ammoniaque
1,64 mole/l d'hexaméthylènetétramine

L'appareil de gélification du « sol » est constitué par une colonne en verre de 80 mm de diamètre et de 1,50 m de haut, prolongée à son sommet par une zone de désengagement de 100 mm de diamètre.

3

# 0 055 652

Le « sol » est injecté à raison de 0,40 l/h au sommet de cette zone de désengagement, au travers d'un tube de diamètre intérieur d'un millimètre, disposé concentriquement à l'intérieur d'un tube de 3 mm de diamètre intérieur, par lequel on injecte, comme fluide moteur, de l'éthyl-2-hexanol à une température de 25 °C, avec un débit de 2,8 l/h. Vers la base de la colonne, on introduit avec un débit de 25 l/h un autre courant d'éthyl-2-hexanol, réchauffé à une température de 120 °C et servant de solvant de déshydratation. Ce courant d'éthyl-2-hexanol parcourt la colonne de bas en haut et sort par un trop plein vers le sommet de la zone de désengagement.

A l'extrémité inférieure de la colonne on recueille les microbilles d'oxyde de chrome. La production de l'appareil est d'environ 60 g/h.

Les microbilles sont lavées abondamment à l'ammoniaque concentrée, puis à l'eau et séchées à 130 °C. Elles sont ensuite activées à 400 °C, pendant 5 heures, sous atmosphère d'air. Le diamètre moyen des microbilles est d'environ 300 µm.

Le catalyseur ainsi obtenu présente les caractéristiques suivantes :

| | |
|---|---|
| — densité : | 2,198 |
| — surface spécifique : | 52,3 m²/g |
| — surface des pores ≥ 250 Å : | 0,05 m²/g |
| — surface des pores 250-50 Å : | 0,765 m²/g |
| — surface des pores 50-40 Å : | 1,61 m²/g |

## Exemple 2

Préparation de microbilles d'oxyde de chrome.

Dans l'appareil décrit à l'exemple 1, on introduit un « sol » formé par le mélange en continu de 0,2 l/h d'une solution aqueuse contenant 4 moles/l de trioxyde de chrome $CrO_3$ et de 0,2 l/h de méthanol. La réduction du trioxyde de chrome est pratiquement immédiate, comme le montre le changement de coloration.

Les autres conditions sont identiques à celles de l'exemple 1.

La production de l'appareil est de 60 g/h de microbilles d'un diamètre de 284 µm.

## Exemple 3

Préparation de microbilles d'oxyde de chrome.

Dans un appareil constitué d'une colonne de 300 mm de diamètre et de 4,50 m de hauteur, on injecte, à l'aide de 18 aiguilles de 1,5 mm de diamètre intérieur, 20 l/h d'un « sol » préparé à partir de :

12 moles de sulfate de chrome
6 moles de sesquioxyde de chrome non calciné, en poudre fine
39,4 moles d'ammoniaque
21,4 moles d'hexaméthylènetétramine
11,0 moles de silice colloïdale LUDOX (marque déposée de la Société du Pont de Nemours).

Dans cet appareil on n'utilise pas de fluide moteur pour entraîner les billes.

L'éthyl-2-hexanol servant de solvant de déshydratation est alimenté au débit de 120 l/h. Sa température est maintenue à 120 °C.

La production est de 4,32 kg/h en microbilles dont le diamètre est compris entre 0,8 et 2 mm.

## Exemple 4

Fluoruration de l'hexachloréthane.

Cet exemple illustre l'utilisation d'un catalyseur selon l'exemple 1 dans la fluoruration de l'hexachloréthane, formé in situ par réaction du chlore sur le tétrachloréthylène.

Dans un réacteur à lit fluidisé, contenant le catalyseur et maintenu à la température de 340 °C, on fait passer à la vitesse de 32 moles/h/l un mélange d'acide fluorhydrique, de chlore et de tétrachloréthylène, dans le rapport molaire 4,26/1,1/1.

Le taux de transformation du tétrachloréthylène est de :

7 % en tétrachlorodifluoréthane
25 % en trichlorotrifluoréthane
57 % en dichlorotétrafluoréthane (contenant 83 % d'isomère symétrique)
10 % en monochloropentafluoréthane.

Le taux de transformation de l'acide fluorhydrique est de 83 %.

4

Si l'on opère dans les mêmes conditions à la température de 304 °C, le taux de transformation du tétrachloréthylène est de :

9 % en tétrachlorodifluoréthane
46 % en trichlorotrifluoréthane
41 % en dichlorotétrafluoréthane (contenant 92 % d'isomère symétrique)
3 % en monochloropentafluoréthane.

## Exemple 5

Fluoruration du dichlorotétrafluoréthane.

Sur un catalyseur identique à celui de l'exemple 1, on fait passer à 400 °C un mélange de dichlorotétrafluoréthane et d'acide fluorhydrique dans le rapport molaire 2,42/1, avec un débit de 8,6 moles/h/l, en présence d'une faible quantité de chlore.
Le taux de transformation de l'acide fluorhydrique est de 52 %.
Le taux de transformation du dichlorotétrafluoréthane est de :

55 % en monochloropentafluoréthane
27 % en hexafluoréthane.

## Exemple 6

Fluoruration du dichlorodifluorométhane.

Sur le catalyseur de l'exemple 1, maintenu à 400 °C, on fait passer un mélange d'acide fluorhydrique et de dichlorodifluorométhane, dans le rapport molaire 2,9/1, à un débit de 17,5 moles/h/l.
Le taux de transformation de l'acide fluorhydrique est de 67 %.
Le taux de transformation du dichlorodifluorométhane est de :

5 % en chlorotrifluorométhane
95 % en tétrafluorométhane.

## Exemple 7

Fluoruration de l'hexachloracétone.

Le catalyseur de l'exemple 1 est utilisé pour la fluoruration de l'hexachloracétone dans les conditions suivantes :

— Température = $300 \pm 50$ °C
— Rapport molaire HF/$Cl_3C$—CO—$CCl_3$ = 7,7/1
— Débit = 4,8 moles/h/l.

On obtient un taux de transformation de l'acide fluorhydrique de 76 %.
Les gaz sortant du réacteur de fluoruration contiennent, en moles, 75 % d'hexafluoracétone, 6 % de chloropentafluoracétone et de faibles quantités de dichlorotétrafluoracétone et de trichlorotrifluoracétone.
Le taux de coupure de l'hexachloracétone de départ est très faible.

## Revendications

1. Catalyseurs de fluoruration en phase gazeuse des dérivés aliphatiques, à base d'oxyde de chrome, caractérisés en ce qu'ils sont constitués de microbilles de $Cr_2O_3$ amorphe, d'un diamètre de 0,1 à 3 mm.

2. Procédé de préparation de catalyseurs selon la revendication 1, par un procédé « sol-gel », caractérisé en ce qu'un « sol » d'hydroxyde de chrome est dispersé sous forme de gouttelettes dans un solvant organique, non miscible ou partiellement miscible à l'eau, et gélifié, et en ce que les microbilles obtenues sont lavées, séchées, puis soumises à un traitement d'activation thermique à des températures de 100 à 500 °C.

3. Procédé selon la revendication 2, caractérisé en ce que le « sol » d'hydroxyde de chrome est obtenu à partir d'une solution aqueuse d'un sel de chrome, à laquelle sont ajoutées de l'ammoniaque et de l'hexaméthylènetétramine.

4. Procédé selon la revendication 2, caractérisé en ce que le « sol » est obtenu par réduction au moyen d'un alcool d'une solution aqueuse de trioxyde de chrome.

5. Procédé selon l'une des revendications 2 à 4, caractérisé en ce que la gélification du « sol » est effectuée au sein de l'éthyl-2-hexanol.

6. Procédé selon l'une des revendications 2 à 5, caractérisé en ce que l'on ajoute au « sol » 1 à 10 % de silice colloïdale par rapport aux matières sèches.

7. Procédé de fluoruration en phase gazeuse des dérivés chlorés aliphatiques caractérisé par l'utilisation dans des réacteurs à lit fluidisé de catalyseurs selon la revendication 1.

### Claims

1. Chromium oxide-based catalysts for gas phase fluorination of aliphatic derivatives, characterised in that they consist of microspherules of amorphous $Cr_2O_3$ of diameter 0.1 to 3 mm.

2. Process for the preparation of catalysts according to Claim 1 by a « sol-gel » process, characterised in that a chromium hydroxide « sol » is dispersed in the form of droplets in an organic solvent which is immiscible or partially miscible with water, and gelified, and in that the microspherules obtained are washed, dried and then subjected to a thermal activation treatment at temperatures from 100 to 500 °C.

3. Process according to Claim 2, characterised in that the chromium hydroxide « sol » is obtained from an aqueous solution of a chromium salt, to which ammonia and hexamethylenetetramine are added.

4. Process according to Claim 2, characterised in that the « sol » is obtained by reduction of an aqueous solution of chromium trioxide by means of an alcohol.

5. Process according to one of Claims 2 to 4, characterised in that the gelification of the « sol » is performed in 2-ethyl-hexanol.

6. Process according to one of Claims 2 to 5, characterised in that 1 to 10 % of colloidal silica, relative to the dry matter, is added to the « sol ».

7. Process for gas phase fluorination of aliphatic chlorinated derivatives, characterised in that catalysts according to claim 1 are used in fluidised-bed reactors.

### Ansprüche

1. Katalysatoren zur Fluorierung von aliphatischen Verbindungen in der Gasphase auf Basis von Chromoxid, dadurch gekennzeichnet, daß sie aus Mikrokugeln von amorphem $Cr_2O_3$ mit einem Durchmesser von 0,1 bis 3 mm bestehen.

2. Verfahren zur Herstellung von Katalysatoren nach Anspruch 1 durch ein « Sol-Gel »-Verfahren, dadurch gekennzeichnet, daß ein « Sol » von Chromhydroxid in Form kleiner Tröpfchen in einem organischen, mit Wasser nicht mischbaren oder teilweise mischbaren Lösungsmittel dispergiert und in ein Gel umgewandelt wird, und daß die erhaltenen Mikrokugeln gewaschen, getrocknet und anschließend einer thermischen Aktivierung bei Temperaturen zwischen 100 und 500 °C unterworfen werden.

3. Verfahren nach Anspruch 2, dadurch gekennzeichnet, daß das « Sol » von Chromhydroxid aus einer wässrigen Lösung eines Chromsalzes erhalten wird, der Ammoniak und Hexamethylentetramin zugesetzt ist.

4. Verfahren nach Anspruch 2, dadurch gekennzeichnet, daß das « Sol » durch Reduktion einer wässrigen Chromtrioxid-Lösung mit einem Alkohol erhalten wird.

5. Verfahren nach einem der Ansprüche 2 bis 4, dadurch gekennzeichnet, daß die Gelifizierung des « Sols » in Ethyl-2-hexanol durchgeführt wird.

6. Verfahren nach einem der Ansprüche 2 bis 5, dadurch gekennzeichnet, daß man dem Sol 1 bis 10 % kolloidales Siliciumdioxid, bezogen auf Trockensubstanz, zusetzt.

7. Verfahren zur Fluorierung von aliphatischen Chlorverbindungen in der Gasphase, gekennzeichnet durch die Verwendung von Katalysatoren nach Anspruch 1 in Fließbettreaktoren.